# EUROPEAN PATENT APPLICATION

(11) **EP 3 957 254 A2**
(43) Date of publication of application: **23.02.2022**
(21) Application number: 21191881.8
(22) Date of filing: 18.08.2021
(51) Int. Cl.: A61B 17/072

(54) **SURGICAL STAPLING DEVICE WITH SLEEVE TO IMPROVE GRASPING CAPABILITY**

(30) Priority: 19.08.2020 US 202016997102
(71) Applicant: Covidien LP, Mansfield, MA 02048 (US)
(72) Inventor: GEORGE, Sabastian, 562125 Bangalore (IN); SHANMUGAVEL, Logamurugaraj, 642007 Pollachi (IN)
(74) Representative: Maschio, Antonio

(57) **Abstract**

A surgical device includes gripping sleeves that can be secured to jaws of the surgical device to allow the device to function as a grasper. The gripping sleeves include nonslip surfaces which are brought into juxtaposed alignment with each other when the surgical device is moved from an open position to a clamped position to clamp tissue between the nonslip surfaces of the gripping sleeves.

## Description

### FIELD

This disclosure is generally related to surgical stapling devices and, more particularly, to a sleeve for use with surgical stapling devices to improve a grasping capability of the surgical stapling device.

### BACKGROUND

Endoscopic surgical procedures are performed through small incisions in a patient and minimize trauma to the patient to shorten patient recovery times. During an endoscopic surgical procedure, a variety of instruments may be inserted into the patient to perform the surgical procedure. These instruments may include stapling devices, graspers, clip appliers or the like. Each time a new surgical instrument is inserted into a patient during an endoscopic surgical procedure, the chance of infection is increased.

Surgical stapling devices for stapling tissue during a surgical procedure are well known. Such devices allow a clinician to suture tissue more quickly than traditional suturing techniques to shorten the length of the surgical procedure and minimize patient trauma. Many endoscopic surgical stapling devices are disposable after a single use or include disposable loading units that are disposable after a single use. In order to reduce the number of surgical instruments inserted into the patient during an endoscopic surgical procedure, a surgical stapling device is sometimes used as grasper manipulate tissue within a body cavity. Grasping capabilities of surgical stapling devices is limited.

A continuing need exists in the art for a device to improve the grasping capabilities of a surgical stapling device.

### SUMMARY

This disclosure is directed to one or more gripping sleeves that can be used to convert a surgical stapling device into a grasping device.

In aspects of this disclosure, a surgical device includes a tool assembly and a first gripping sleeve. The tool assembly includes a first jaw and a second jaw that is movably coupled to the first jaw such that the tool assembly is movable between open and clamped positions. The tool assembly defines a longitudinal axis. The first gripping sleeve has a tubular body that defines a bore and includes a nonslip surface. The first gripping sleeve is received about the first jaw such that the nonslip surface is in juxtaposed alignment with the second jaw when the tool assembly is in the clamped position.

In still other aspects of the disclosure, a tool assembly includes a first jaw that supports a staple cartridge and a second jaw that supports an anvil. The second jaw is movably coupled to the first jaw such that the tool assembly is movable between open and clamped positions. The tool assembly defines a longitudinal axis. The first gripping sleeve has a resilient tubular body that defines a bore and includes a nonslip surface. The first gripping sleeve is received about the first jaw such that the nonslip surface is in juxtaposed alignment with the second jaw when the tool assembly is in the clamped position. The second gripping sleeve has a resilient tubular body that defines a bore and includes a nonslip surface. The second gripping sleeve is received about the second jaw such that the nonslip surface of the second gripping sleeve is in juxtaposed alignment with the nonslip surface of the first gripping sleeve when the tool assembly is in the clamped position.

In aspects of the disclosure, the surgical device includes a second gripping sleeve that has a tubular body that defines a bore and includes a nonslip surface.

In some aspects of the disclosure, the second gripping sleeve is received about the second jaw such that the nonslip surface of the second gripping sleeve is in juxtaposed alignment with the nonslip surface of the first gripping sleeve when the tool assembly is in the clamped position.

In certain aspects of the disclosure, the nonslip surfaces of the first and second gripping sleeves include protrusions that are positioned along the nonslip surfaces.

In aspects of the disclosure, the protrusions include ribs positioned along the nonslip surfaces of the first and second gripping sleeves in a direction transverse to the longitudinal axis of the tool assembly.

In some aspects of the disclosure, the first gripping sleeve is formed from a resilient material.

In certain aspects of the disclosure, the bore defines a diameter that is smaller than a diameter of the first jaw such that the first gripping sleeve must be stretched about the first jaw.

In some aspects of the disclosure, the first jaw supports a staple cartridge and the second jaw supports an anvil.

In certain aspects of the disclosure, the surgical device includes a handle assembly and an elongate body having a proximal portion and a distal portion, and the handle assembly is coupled to the proximal portion of the elongate body and the tool assembly is coupled to the distal portion of the elongate body.

In aspects of the disclosure, the tool assembly forms part of a reload assembly that includes a proximal body portion, the proximal body portion that is releasably coupled to the elongate body.

In some aspects of the disclosure, the first and second gripping sleeves are removably secured to the first and second jaws, respectively, of the tool assembly.

In certain aspects of the disclosure, the nonslip surfaces extend along the entire length of the first and second gripping sleeves.

In other aspects of the disclosure, a kit includes a surgical device and a first gripping sleeve. The surgical device includes a tool assembly that defines a longitudinal axis. The tool assembly has a first jaw and a second jaw that is movably coupled to the first jaw such that the tool assembly is movable between open and clamped positions. The first gripping sleeve has a tubular body that defines a bore and includes a nonslip surface. The bore is dimensioned to allow the tubular body to be removably received about the first jaw such that the nonslip surface is in juxtaposed alignment with the second jaw when the tool assembly is in the clamped position.

In aspects of the disclosure, the kit includes a second gripping sleeve that has a tubular body that defines a bore and includes a nonslip surface. The bore defined by the second gripping sleeve is dimensioned to allow the tubular body to be removably received about the second jaw of the tool assembly such that the nonslip surface of the second gripping sleeve is in juxtaposed alignment with the nonslip surface of the first gripping sleeve when the tool assembly is in the clamped position.

Other features of the disclosure will be appreciated from the following description.

### BRIEF DESCRIPTION OF DRAWINGS

Various aspects of the disclosure are described herein below with reference to the drawings, wherein:
FIG. 1 is a perspective view of a surgical stapling device including a tool assembly in an open position with grasping sleeves according to aspects of the disclosure supported on jaws of the tool assembly;
FIG. 2 is a side perspective view of the tool assembly and grasping sleeves shown in FIG. 1 with the grasping sleeves separated from the first and second jaws;
FIG. 3 is a side perspective view of the grasping sleeve of the first jaw of the tool assembly shown in FIG. 2;
FIG. 4 is a side perspective view of the grasping sleeve of the second jaw of the tool assembly shown in FIG. 2;
FIG. 5 is an enlarged view of the indicated area of detail shown in FIG. 1;
FIG. 6 is a side perspective view of the tool assembly shown in FIG. 1 with the grasping sleeves secured to jaws of the tool assembly and the tool assembly in the open position;
FIG. 7 is a cross-sectional view taken through section line 7-7 of FIG. 6;
FIG. 8 is a cross-sectional view taken through section line 8-8 of FIG. 6;
FIG. 9 is a side perspective view of the tool assembly of the surgical stapling device shown in FIG. 1 with the grasping sleeves supported on the jaws of the tool assembly and the tool assembly in a clamped position; and
FIG. 10 is a side perspective view of the surgical stapling device shown in FIG. 1 inserted into a body cavity through a cannula with the grasping sleeves supported on the jaws of the tool assembly and the tool assembly clamped about a body vessel.

### DETAILED DESCRIPTION

The disclosed surgical stapling device and grasping sleeves will now be described in detail with reference to the drawings in which like reference numerals designate identical or corresponding elements in each of the several views. However, it is to be understood that the aspects of the disclosure are merely exemplary of the disclosure and may be embodied in various forms. Well-known functions or constructions are not described in detail to avoid obscuring the disclosure in unnecessary detail. Therefore, specific structural and functional details disclosed herein are not to be interpreted as limiting, but merely as a basis for the claims and as a representative basis for teaching one skilled in the art to variously employ the disclosure in virtually any appropriately detailed structure. In addition, directional terms such as front, rear, upper, lower, top, bottom, distal, proximal, and similar terms are used to assist in understanding the description and are not intended to limit the disclosure.

In this description, the term "proximal" is used generally to refer to that portion of the device that is closer to a clinician, while the term "distal" is used generally to refer to that portion of the device that is farther from the clinician. In addition, the term "endoscopic" is used generally used to refer to endoscopic, laparoscopic, arthroscopic, and/or any other procedure conducted through a small diameter incision or cannula. Further, the term "clinician" is used generally to refer to medical personnel including doctors, nurses, and support personnel.

This disclosure is directed to gripping sleeves that can be secured to jaws of a surgical device to allow the device to function as a grasper. In aspects of the disclosure, the gripping sleeves include nonslip surfaces which are brought into juxtaposed alignment with each other when the surgical device is moved from an open position to a clamped position to clamp tissue between the nonslip surfaces of the gripping sleeves. In other aspects of the disclosure, the gripping sleeves are received about the jaws of the surgical device and are formed of a resilient material to retain the gripping sleeves on the first and second jaws.

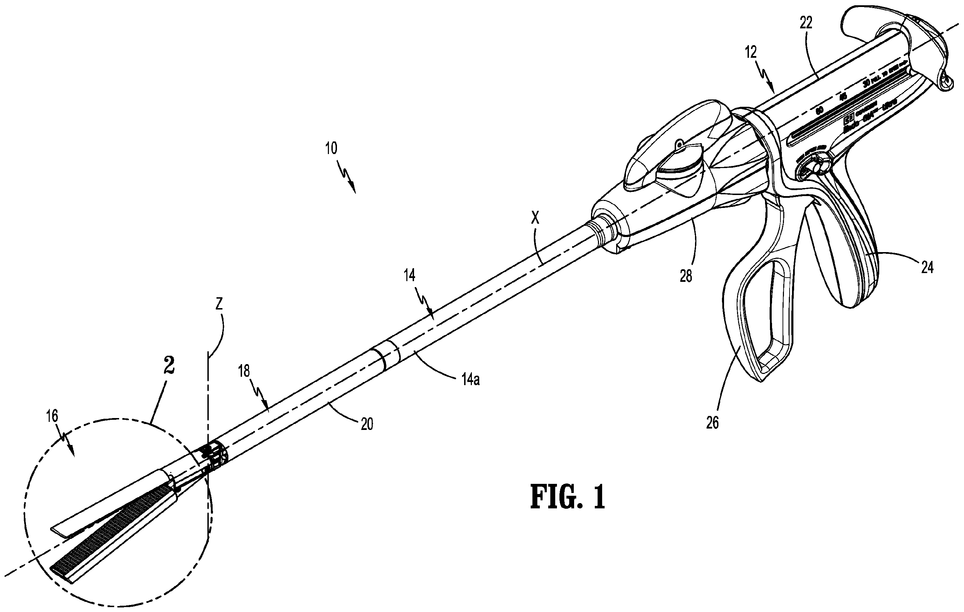

FIG. 1 illustrates the disclosed surgical stapling device shown generally as stapling device 10 which includes a handle assembly 12, an elongate body 14, and a tool assembly 16. Although not described in detail herein, the tool assembly 16 can form part of a reload assembly 18 that is releasably coupled to the elongate body 14 and includes a proximal body portion 20 and the tool assembly 16. Alternately, the tool assembly 16 can be secured directly to a distal portion 14a of the elongate body 14. The elongate body 14 defines a longitudinal axis "X".

The handle assembly 12 includes a body 18 that defines a hand grip 24, a trigger 26 that is mounted on the body 18 and is movable towards the hand grip 24, and a rotation knob 22. The rotation knob 22 is rotatably supported on a distal portion of the body 22 of the handle assembly 12 and supports the elongate body 14 to facilitate rotation of the elongate body 14 and the tool assembly 16 in relation to the handle assembly 12 about the longitudinal axis "X". The trigger 26 is manually operable to control operation of the various functions of the stapling device 10 including approximation, firing and cutting. In aspects of the disclosure, the handle assembly 12 also supports an articulation knob 30 which is operable to pivot the tool assembly 16 about a pivot member defining an axis "Z" that is transverse to the longitudinal axis "X" of the elongate body 14. Although the stapling device 10 is illustrated as a manually operated stapling device, it is envisioned that aspects of this disclosure are suitable for use with robotically controlled stapling devices and electrically powered stapling devices. U.S. Patent No. 9,055,943 ("the '943 Patent") discloses a surgical stapling device including an electrically powered handle assembly and U.S. Patent No. 6,241,139 ("the '139 Patent") discloses a manually actuated handle assembly. For a more detailed description of construction and operation of an exemplary handle assembly and elongate body 14 which are suitable for use with the tool assembly 16 of the stapling device 10, see, e.g., the '943 and '139 Patents.

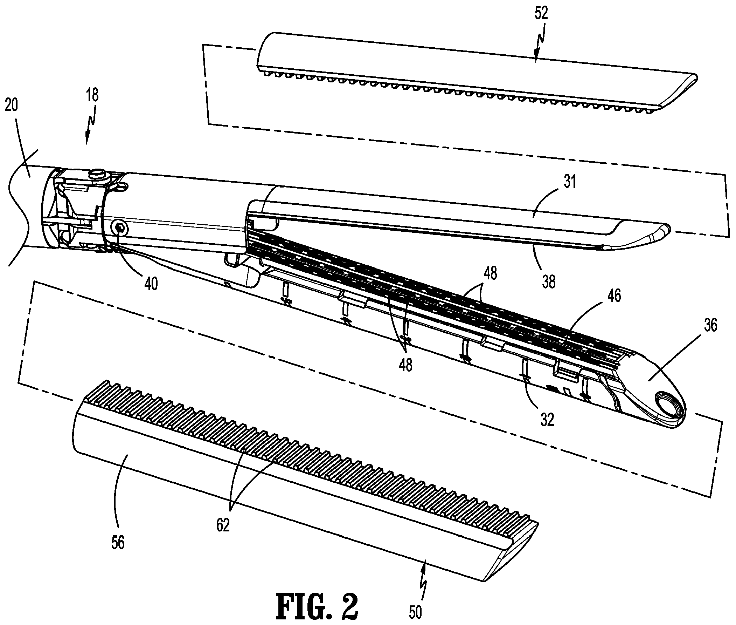

FIG. 2 illustrates the tool assembly 16 of the stapling device 10 (FIG. 1). The tool assembly 16 includes first and second jaws 32 and 34. In aspects of the disclosure, the first jaw 32 supports a staple cartridge 36 and the second jaw 34 supports an anvil 38. The first jaw 32 is movable in relation to the second jaw 34 such that the tool assembly 16 can move between open and clamped positions. In the clamped position, the staple cartridge 36 is in juxtaposed alignment with the anvil 38. In aspects of the disclosure, the first jaw 32 is coupled to the second jaw 34 by pivot members 40 such that the first jaw 32 can pivot in relation to the second jaw 34 and in relation to the proximal body portion 20 of the reload assembly 18 between the open and clamped positions. Alternately, it is envisioned that the first jaw 32 could pivot in relation to the second jaw 34 and in relation to the proximal body portion 20 of the reload assembly 18 to move the tool assembly 16 between the open and clamped positions.

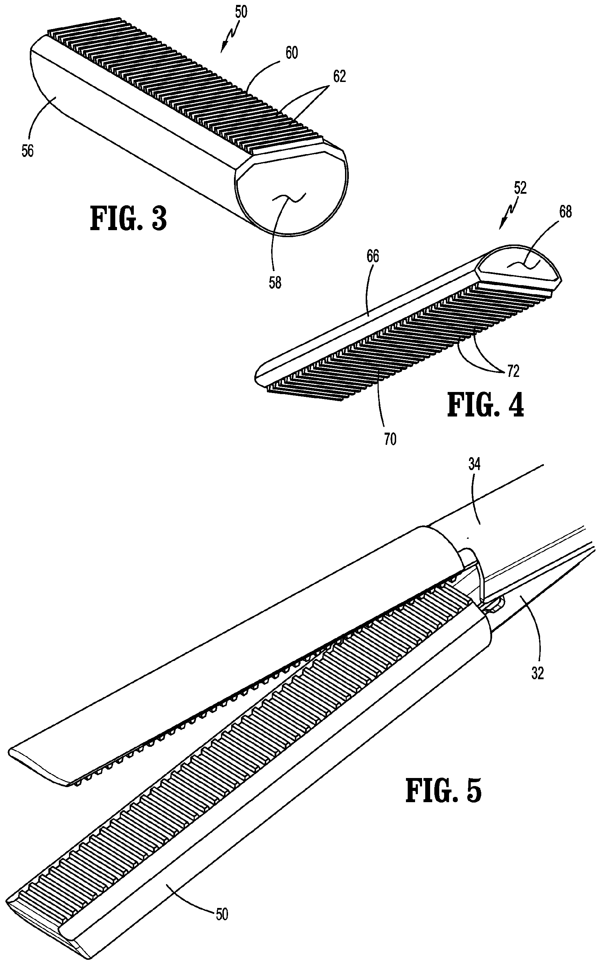

The staple cartridge 36 (FIG. 4) is received within a cavity defined by the first jaw 32 and includes a cartridge body 44 that defines a central knife slot 46 and staple receiving pockets 48 which are aligned in rows on each side of the central knife slot 46. Each of the staple receiving pockets 48 receives a staple (not shown) and a pusher (not shown). The cartridge body 44 supports an actuation sled (not shown) that is movable through the cartridge body 44 upon actuation of the stapling device 10 (FIG. 1) to eject staples from the cartridge body 44 into the anvil 38 when the tool assembly 16 is in its clamped position. In aspects of the disclosure, the staple cartridge 36 is removable from the first jaw 32 and replaceable to facilitate reuse of the stapling device 10 (FIG. 1). The first jaw 32 supports a first gripping sleeve 50 and the second jaw 34 supports a second gripping sleeve 52. The '139 Patent describes interaction of the actuation sled and pushers to eject staples from the staple cartridge.

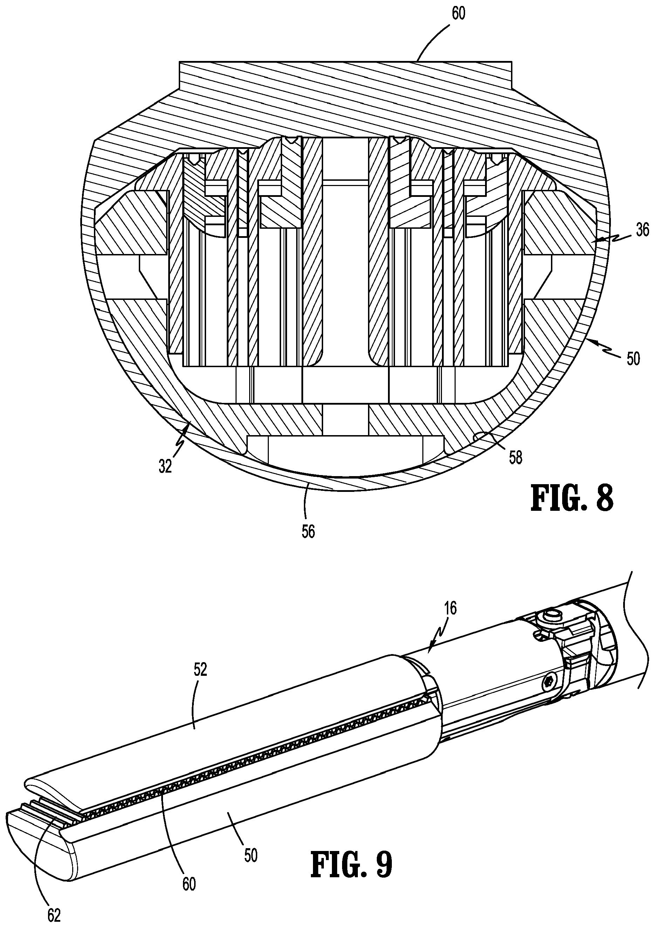

FIG. 3 illustrates the first gripping sleeve 50 which includes a tubular body 56 that defines a bore 58 (FIG. 5) that receives the first jaw 32 and the staple cartridge 36 (FIG. 8). Although the bore 58 is illustrated to be a through bore, it is envisioned that a distal end of the bore 58 could be closed. In aspects of the disclosure, the tubular body 56 of the first gripping sleeve 50 includes a nonslip surface 60 that extends along a least a portion of the longitudinal length of the first gripping sleeve 50. In some aspects of the disclosure, the nonslip surface 60 extends along substantially the entire length of the first gripping sleeve 50 and includes transverse ribs 62 that are spaced along at least a portion of the length of the nonslip surface 60. Although the ribs 62 are shown to be uniformly distributed along the entire length of the nonslip surface 60, it is envisioned that the ribs 62 could be provided in groups or be further spaced along the nonslip surface 60. Alternately, it is envisioned that the nonslip surface 60 can be formed or defined by a variety of different patterns of raised surfaces or recesses, or of slip resistant materials that prevent slippage along the surface. In some aspects of the disclosure, the first gripping sleeve 50 is formed of a resilient material such as an elastomer that can be stretched about the first jaw 32 and the staple cartridge 36 (FIG. 8) to secure the first gripping sleeve 50 to the first jaw 32.

In aspects of the disclosure, the portion of the first gripping sleeve 50 that supports or includes the nonslip surface 60 has a thickness that is greater than the thickness of the remaining portion of the tubular body 56 of the first gripping sleeve 50. The reduced thickness of the remaining portion of the tubular body 56 of the first gripping sleeve 50 allows the first gripping sleeve 50 to be stretched about the first jaw 32 to secure the first gripping sleeve 50 to the first jaw 32.

FIG. 4 illustrates the second gripping sleeve 52 which includes a tubular body 66 that defines a bore 68 that receives the second jaw 34 and the anvil 38. Although the bore 68 is illustrated to be a through bore, it is envisioned that a distal end of the bore 68 could be closed. In aspects of the disclosure, the tubular body 66 of the second gripping sleeve 52 also includes a nonslip surface 70 that extends along a least a portion of the longitudinal length of the second gripping sleeve 52. In some aspects of the disclosure, the nonslip surface 70 extends along substantially the entire length of the second gripping sleeve 52 and includes transverse ribs 72 that are spaced along at least a portion of the length of the nonslip surface 70. Alternately, it is envisioned that the nonslip surface 70 can be formed or defined by a variety of different patterns of raised surfaces or recesses, or slip-resistant materials that prevent slippage along the surface. In some aspects of the disclosure, the second gripping sleeve 52 is formed of a resilient material such as an elastomer that can be stretched about the second jaw 34 and the anvil 38 to secure the second gripping sleeve 52 to the second jaw 34.

In aspects of the disclosure, the portion of the second gripping sleeve 52 that supports or includes the nonslip surface 70 has a thickness that is greater than the thickness of the remaining portion of the tubular body 66 of the second gripping sleeve 52. The reduced thickness of the remaining portion of the tubular body 66 of the second gripping sleeve 52 allows the second gripping sleeve 52 to be stretched about the second jaw 34 to secure the second gripping sleeve 52 to the first jaw 34.

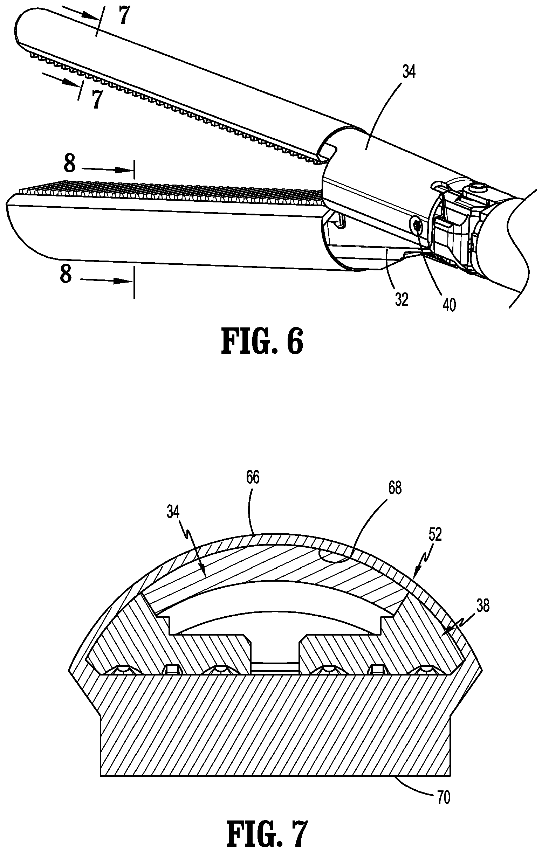

FIGS. 6-9 illustrate the tool assembly 16 with the first gripping sleeve 50 received about the first jaw 32 and the staple cartridge 36 and the second gripping sleeve 52 received about the second jaw 34 and the anvil 38. When the first and second gripping sleeves 50 and 52 are received about the first and second jaws 32 and 34, respectively and the tool assembly 16 is moved to the clamped position (FIG. 9), the nonslip surfaces 60 and 70 of the first and second gripping sleeves 50 and 52 are in juxtaposed alignment. In aspects of the disclosure, the nonslip surfaces 60 and 70 of the first and second gripping sleeves 50 and 52, respectively, are positioned to be engaged with each other when the tool assembly 16 is in the clamped position. Alternately, the thickness of the first and second gripping sleeves can be dimensioned to provide some gap between the first and second sleeves 50 and 52 when the tool assembly 16 is in the clamped position.

In aspects of the disclosure, the nonslip surfaces 60 and 70 of the first and second gripping sleeves 50 and 52 are integrally formed with the first and second gripping sleeves 50 and 52. Alternately, the nonslip surfaces 60 and 70 can be secured to the first and second gripping sleeves 50 and 52 using adhesives or laminates, or the like.

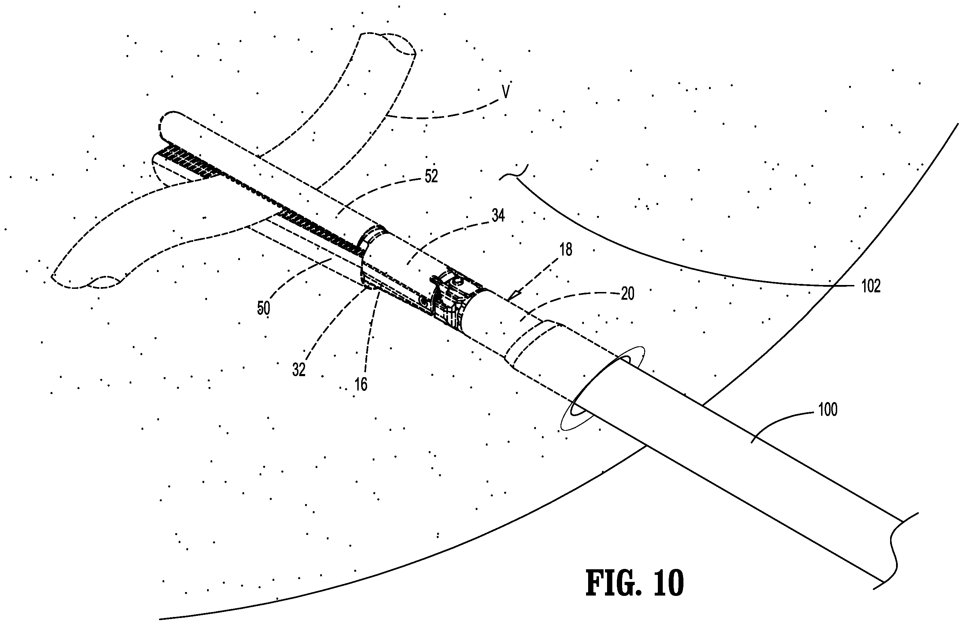

FIG. 10 illustrates the distal portion of the stapling device 10 (FIG. 1) inserted through a cannula 100 into a body cavity 102 and gripping a vessel "V". The first and second gripping sleeves 50 and 52 are supported on the first and second jaws 32 and 34, respectively. In order to position the stapling device 10 (FIG. 1) about the vessel "V", the tool assembly 16 is moved to the open position and the vessel "V" is positioned between the nonslip surfaces 60 and 70 of the first and second gripping sleeves 50 and 52. The tool assembly 16 is then moved to the clamped position to compress the vessel "V" between the nonslip surfaces 60 and 70 of the first and second gripping sleeves 50 and 52.

It is envisioned that the first and second gripping sleeves 50 and 52 can be used together or independently to grip tissue. It is also envisioned that the first and second gripping sleeves 50 and 52 can be secured to a one or both of the jaws of the stapling device prior to or before firing of the stapling device to adapt the stapling device for grasping of a vessel "V". In some aspects of the disclosure, one or both of the gripping sleeves 50 and 52 are coupled to one or both of the jaws 32 and 34 of the tool assembly 16 after the stapling device 10 (FIG. 1) is fired to allow the stapling device 10 to function as a grasper after the stapling device performs its stapling function.

It is envisioned that the stapling device 10 (FIG. 1) can be provided in a kit with one or both of the first or second gripping sleeves. Thus, after a clinician has completed a stapling function during a surgical procedure, one or both of the first or second gripping sleeves can be secured to one or both of the jaws of the stapling device 10 to convert the stapling device 10 into a grasper. It is also envisioned that an internal surface of the first and/or second gripping sleeves can be formed with a tacky or slip resistant surface that is configured to retain the gripping sleeves on the first and/or second jaws of the stapling device.

Persons skilled in the art will understand that the devices and methods specifically described herein and illustrated in the accompanying drawings are nonlimiting exemplary aspects of the disclosure. It is envisioned that the elements and features illustrated or described in connection with one exemplary embodiment may be combined with the elements and features of another without departing from the scope of the present disclosure. As well, one skilled in the art will appreciate further features and advantages of the disclosure based on the above-described aspects of the disclosure. Accordingly, the disclosure is not to be limited by what has been particularly shown and described, except as indicated by the appended claims.

The invention may be described by reference to the following numbered paragraphs:-
1. A surgical device comprising:
   a tool assembly including a first jaw and a second jaw movably coupled to the first jaw such that the tool assembly is movable between open and clamped positions, the tool assembly defining a longitudinal axis; and
   a first gripping sleeve having a tubular body defining a bore and including a nonslip surface, wherein the first gripping sleeve is received about the first jaw such that the nonslip surface is in juxtaposed alignment with the second jaw when the tool assembly is in the clamped position.
2. The surgical device of paragraph 1, further including a second gripping sleeve having a tubular body defining a bore and including a nonslip surface, wherein the second gripping sleeve is received about the second jaw such that the nonslip surface of the second gripping sleeve is in juxtaposed alignment with the nonslip surface of the first gripping sleeve when the tool assembly is in the clamped position.
3. The surgical device of paragraph 1, wherein the nonslip surfaces of the first and second gripping sleeves include protrusions that are positioned along the nonslip surfaces.
4. The surgical device of paragraph 3, wherein the protrusions include ribs positioned along the nonslip surfaces of the first and second gripping sleeves in a direction transverse to the longitudinal axis of the tool assembly.
5. The surgical device of paragraph 1, wherein the first gripping sleeve is formed from a resilient material.
6. The surgical device of paragraph 5, wherein the bore defines a diameter that is smaller than a diameter of the first jaw such that the first gripping sleeve must be stretched about the first jaw.
7. The surgical device of paragraph 2, wherein the first jaw supports a staple cartridge and the second jaw supports an anvil.
8. The surgical device of paragraph 7, further including a handle assembly and an elongate body having a proximal portion and a distal portion, the handle assembly coupled to the proximal portion of the elongate body and the tool assembly coupled to the distal portion of the elongate body.
9. The surgical device of paragraph 8, wherein the tool assembly forms part of a reload assembly, the reload assembly including a proximal body portion, the proximal body portion being releasably coupled to the elongate body.
10. The surgical device of paragraph 2, wherein the first and second gripping sleeves are removably secured to the first and second jaws, respectively, of the tool assembly.
11. The surgical device of paragraph 1, wherein the nonslip surface extends along the entire length of the first gripping sleeve.
12. A kit comprising:
   a surgical device including a tool assembly a longitudinal axis, the tool assembly having a first jaw and a second jaw movably coupled to the first jaw such that the tool assembly is movable between open and clamped positions; and
   a first gripping sleeve having a tubular body defining a bore and including a nonslip surface, the bore dimensioned to allow the tubular body to be removably received about the first jaw such that the nonslip surface is in juxtaposed alignment with the second jaw when the tool assembly is in the clamped position.
13. The kit of paragraph 12, further including a second gripping sleeve having a tubular body defining a bore and including a nonslip surface, the bore defined by the second gripping sleeve dimensioned to allow the tubular body to be removably received about the second jaw such that the nonslip surface of the second gripping sleeve is in juxtaposed alignment with the nonslip surface of the first jaw when the tool assembly is in the clamped position.
14. The kit of paragraph 12, wherein the nonslip surfaces of the first and second gripping sleeves include protrusions that are positioned along the nonslip surfaces.
15. The kit of paragraph 12, wherein the first gripping sleeve is formed from a resilient material.
16. The kit of paragraph 12, wherein the first jaw of the stapling device supports a staple cartridge and the second jaw of the stapling device supports an anvil.
17. The kit of paragraph 12, wherein the surgical device includes a handle assembly and an elongate body having a proximal portion and a distal portion, the handle assembly coupled to the proximal portion of the elongate body and the tool assembly coupled to the distal portion of the elongate body.
18. The kit of paragraph 17, wherein the tool assembly of the surgical device forms part of a reload assembly, the reload assembly including a proximal body portion, the proximal body portion being releasably coupled to the elongate body.
19. A surgical device comprising:
   a tool assembly including a first jaw supporting a staple cartridge and a second jaw supporting an anvil, the second jaw movably coupled to the first jaw such that the tool assembly is movable between open and clamped positions, the tool assembly defining a longitudinal axis;
   a first gripping sleeve having a resilient tubular body defining a bore and including a nonslip surface, wherein the first gripping sleeve is received about the first jaw such that the nonslip surface is in juxtaposed alignment with the second jaw when the tool assembly is in the clamped position; and
   a second gripping sleeve having a resilient tubular body defining a bore and including a nonslip surface, wherein the second gripping sleeve is received about the second jaw such that the nonslip surface of the second gripping sleeve is in juxtaposed alignment with the nonslip surface of the first gripping sleeve when the tool assembly is in the clamped position.
20. The surgical device of paragraph 19, wherein the nonslip surfaces of the first and second gripping sleeves include protrusions that are positioned along the nonslip surfaces.

## Claims

1. A surgical device comprising:
a tool assembly including a first jaw and a second jaw movably coupled to the first jaw such that the tool assembly is movable between open and clamped positions, the tool assembly defining a longitudinal axis; and
a first gripping sleeve having a tubular body defining a bore and including a nonslip surface, wherein the first gripping sleeve is received about the first jaw such that the nonslip surface is in juxtaposed alignment with the second jaw when the tool assembly is in the clamped position.

2. The surgical device of claim 1, further including a second gripping sleeve having a tubular body defining a bore and including a nonslip surface, wherein the second gripping sleeve is received about the second jaw such that the nonslip surface of the second gripping sleeve is in juxtaposed alignment with the nonslip surface of the first gripping sleeve when the tool assembly is in the clamped position.

3. The surgical device of claim 1 or claim 2, wherein the nonslip surfaces of the first and second gripping sleeves include protrusions that are positioned along the nonslip surfaces.

4. The surgical device of claim 3, wherein the protrusions include ribs positioned along the nonslip surfaces of the first and second gripping sleeves in a direction transverse to the longitudinal axis of the tool assembly.

5. The surgical device of any preceding claim, wherein the first gripping sleeve is formed from a resilient material; preferably wherein the bore defines a diameter that is smaller than a diameter of the first jaw such that the first gripping sleeve must be stretched about the first jaw.

6. The surgical device of claim 2, wherein the first jaw supports a staple cartridge and the second jaw supports an anvil; preferably further including a handle assembly and an elongate body having a proximal portion and a distal portion, the handle assembly coupled to the proximal portion of the elongate body and the tool assembly coupled to the distal portion of the elongate body.

7. The surgical device of claim 6, wherein the tool assembly forms part of a reload assembly, the reload assembly including a proximal body portion, the proximal body portion being releasably coupled to the elongate body.

8. The surgical device of claim 2, wherein the first and second gripping sleeves are removably secured to the first and second jaws, respectively, of the tool assembly.

9. The surgical device of any preceding claim, wherein the nonslip surface extends along the entire length of the first gripping sleeve.

10. A kit comprising:
a surgical device including a tool assembly a longitudinal axis, the tool assembly having a first jaw and a second jaw movably coupled to the first jaw such that the tool assembly is movable between open and clamped positions; and
a first gripping sleeve having a tubular body defining a bore and including a nonslip surface, the bore dimensioned to allow the tubular body to be removably received about the first jaw such that the nonslip surface is in juxtaposed alignment with the second jaw when the tool assembly is in the clamped position.

11. The kit of claim 10, further including a second gripping sleeve having a tubular body defining a bore and including a nonslip surface, the bore defined by the second gripping sleeve dimensioned to allow the tubular body to be removably received about the second jaw such that the nonslip surface of the second gripping sleeve is in juxtaposed alignment with the nonslip surface of the first jaw when the tool assembly is in the clamped position.

12. The kit of claim 10 or claim 11, wherein the nonslip surfaces of the first and second gripping sleeves include protrusions that are positioned along the nonslip surfaces; and/or wherein the first gripping sleeve is formed from a resilient material.

13. The kit of any of claims 10 to 12, wherein the first jaw of the stapling device supports a staple cartridge and the second jaw of the stapling device supports an anvil.

14. The kit of any of claims 10 to 13, wherein the surgical device includes a handle assembly and an elongate body having a proximal portion and a distal portion, the handle assembly coupled to the proximal portion of the elongate body and the tool assembly coupled to the distal portion of the elongate body; preferably wherein the tool assembly of the surgical device forms part of a reload assembly, the reload assembly including a proximal body portion, the proximal body portion being releasably coupled to the elongate body.

15. A surgical device comprising:
a tool assembly including a first jaw supporting a staple cartridge and a second jaw supporting an anvil, the second jaw movably coupled to the first jaw such that the tool assembly is movable between open and clamped positions, the tool assembly defining a longitudinal axis;
a first gripping sleeve having a resilient tubular body defining a bore and including a nonslip surface, wherein the first gripping sleeve is received about the first jaw such that the nonslip surface is in juxtaposed alignment with the second jaw when the tool assembly is in the clamped position; and
a second gripping sleeve having a resilient tubular body defining a bore and including a nonslip surface, wherein the second gripping sleeve is received about the second jaw such that the nonslip surface of the second gripping sleeve is in juxtaposed alignment with the nonslip surface of the first gripping sleeve when the tool assembly is in the clamped position; preferably wherein the nonslip surfaces of the first and second gripping sleeves include protrusions that are positioned along the nonslip surfaces.
